# FASCICULE DE BREVET EUROPEEN

(11) **EP 1 189 872 B1**
(45) Date de publication et mention de la délivrance du brevet: **30.08.2006**
(21) Numéro de dépôt: 00949626.6
(22) Date de dépôt: 04.07.2000
(51) Int. Cl.: C07C 209/74

(54) **PERFECTIONNEMENT A L'ECHANGE ET A LA LIBERATION D'UNE AMINE, DE SON FLUORURE DE CARBAMOYLE**
VERFAHREN ZUM AUSTAUSCH UND ZUR FREISETZUNG VON AMINEN AUS IHREM CARBAMOYLFLUORID
IMPROVEMENT TO THE EXCHANGE AND RELEASE OF AN AMINE FROM ITS CARBAMOYL FLUORIDE

(30) Priorité: 05.07.1999 FR 9908645
(43) Date de publication de la demande: 27.03.2002
(73) Titulaire: RHODIA CHIMIE, 92512 Boulogne Billancourt Cedex (FR)
(72) Inventeur: GUIDOT, Gilbert, F-30350 Massanes (FR); ROCHIN, Christophe, Princeton, NJ 08540 (US); SAINT-JALMES, Laurent, F-69330 Meyzieu (FR)
(74) Mandataire: Dutruc-Rosset, Marie-Claude
(86) Numéro de dépôt international: PCT/FR2000/001911
(87) Numéro de publication internationale: WO 2001/002337

(56) Documents cités:
- EP-A- 0 129 214
- EP-A- 0 152 310
- EP-A- 0 639 556
- DE-B- 1 138 391

## Description

La présente invention a pour objet un procédé de synthèse de composés comportant à la fois un carbone perfluoré et une fonction aniline à partir de son fluorure de carbamoyle ou de l'isocyanate correspondant et comportant des atomes de chlore à la place des atomes de fluor sur le carbone perfluoré.

Le carbone perfluoré est dans ce cas un carbone de nature aliphatique, c'est-à-dire qu'il est d'hybridation sp³.

La présente invention vise, plus particulièrement, un procédé d'échange et d'hydrolyse dans un même milieu réactionnel (c'est-à-dire sans isolement intermédiaire), notamment à base d'acide fluorhydrique, et souvent dans un même réacteur.

Au cours des derniers lustres et plus précisément de la dernière décennie, les composés comportant un atome aliphatique perfluoré sont devenus de plus en plus importants dans le domaine de l'agrochimie et de la pharmacie. Ces produits perfluorés, le plus souvent comportant un radical perfluorométhyle ou perfluoroéthyle, ont en effet des propriétés physiologiques qui rendent les molécules qui les comportent particulièrement actives.

Aussi a-t-on vu fleurir de nombreuses propositions de procédé conduisant à de tels produits. Le plus souvent, l'agent de fluoration est l'acide fluorhydrique liquide et le substrat de départ est un isocyanate.

On peut ainsi citer les brevets Occidental Chemical Corporation n° EP-A-129 214 et le brevet du prédécesseur en droit de la Demanderesse, à savoir le brevet européen déposé au nom de Rhône-Poulenc Spécialités Chimiques sous le n° 152 310. Plus récemment, un brevet européen au nom de la société Hoechst AG a été publié sous le n° 639 556.

Ces documents exposent différentes variantes de traitement en voie acide fluorhydrique.

Selon cette technique, on commence par protéger la fonction amine par une fonction isocyanate, par exemple par phosgénation. Puis, on chlore en général de manière radicalaire le carbone qui devra être à l'étape finale sous une forme perfluorée. Enfin, on soumet le composé chloré ainsi obtenu à une étape d'échange chlore/fluor dans un milieu acide fluorhydrique liquide anhydre.

Deux variantes ont été à ce jour explorées : la libération de l'amine au moyen d'un chauffage en présence d'un large excès d'acide fluorhydrique (anhydre, bien sûr) pour donner du fluorophosgène ou bien une hydrolyse en milieu acide fluorhydrique par une quantité relativement peu importante d'eau.

La technique utilisant la décomposition des fluorures de carbamoyle en fluorophosgène présente l'inconvénient certain d'être concomitant au dégagement du fluorophosgène dont la toxicité est réputée bien supérieure à celle du phosgène proprement dit qui fut utilisé comme gaz de combat lors de la Première Guerre Mondiale.

Un autre inconvénient de cette technique réside dans la consommation accrue d'acide fluorhydrique, qui est un réactif relativement coûteux dès lors qu'il doit être utilisé en large excès.

Les autres techniques, à savoir les techniques utilisant l'hydrolyse in situ du fluorure de carbamoyle, donnent des rendements qui sont loin d'être excellents.

Ces faibles rendements grèvent lourdement le prix de revient du produit final, et donc la rentabilité de l'opération complète.

Par ailleurs, l'utilisation de très larges excès d'acide fluorhydrique, ce qui implique un encours et un recyclage pour le moins coûteux ainsi qu'une déshydratation subséquente, est aussi extrêmement pénalisante en ce qui concerne le prix de revient de l'opération.

Enfin, au cours de l'étude qui a mené à la présente invention, il a été montré que même lorsque le noyau aromatique de la molécule était appauvri en électrons, la réactivité des fluorures de carbamoyle était très importante et conduisait à de multiples sous-produits qui nuisaient au rendement de transformation, c'est-à-dire à la sélectivité de la transformation.

C'est pourquoi un des buts de la présente invention est de fournir un procédé d'obtention d'aniline, comportant en outre un atome de carbone perfluoré, à partir de l'isocyanate ou du fluorure de carbamoyle correspondant, qui permette d'obtenir des rendements élevés, c'est-à-dire présentant un rendement de transformation (RT) au moins égal à 75%, de préférence à 80%, plus préférentiellement à 90%.

Un autre but de la présente invention est de fournir un procédé du type précédent qui permette d'avoir un taux de transformation (TT) au moins égal à 80%, de préférence au moins égal à 90%.

Un autre but de la présente invention est de fournir un procédé du type précédent qui permette d'avoir un rendement de réaction (RR = TT x RT) au moins égal à 80%, de préférence au moins égal à 90%.

Un autre but de la présente invention est de fournir un procédé du type précédent qui permette d'obtenir un bon rendement avec une quantité minimale d'acide fluorhydrique.

Un autre but de la présente invention est de fournir un procédé du type précédent qui permette d'éviter le dégagement, ou du moins de limiter le dégagement de fluorophosgène.

Ces buts, et d'autres qui apparaîtront par la suite, sont atteints au moyen d'un procédé de synthèse de composés comportant à la fois un carbone perfluoré et une fonction aniline dans lequel on traite un fluorure de carbamoyle arylique, comportant dans sa molécule au moins un carbone aliphatique perhalogéné en position proche d'un doublet réputé stabiliser les carbocations et lequel porte au moins 1, avantageusement au moins 2 atomes d'halogène à échanger avec le fluor, par mise en contact dudit fluorure de carbamoyle arylique avec de l'acide fluorhydrique et de l'eau en phase liquide, caractérisé par le fait qu'il comporte l'étape de mise en contact dudit fluorure de carbamoyle avec de l'acide fluorhydrique, le rapport acide fluorhydrique sur fluorure de carbamoyle étant maintenu pendant la réaction à une valeur au moins égale à 4, de préférence à 5, et que ledit rapport est au plus égal à 11 et par le fait que l'addition de l'eau au mélange réactionnel n'intervient qu'après qu'il ne reste qu'au plus un seul halogène par molécule de substrat à échanger avec le fluor

Ainsi, selon l'invention, le rapport entre le fluorure de carbamoyle et l'acide fluorhydrique (HF/substrat) est maintenu pendant la durée de la réaction à une valeur au moins égale à environ 4, avantageusement à 4,0 ; de préférence à 5, plus préférentiellement à 6. Ledit rapport est avantageusement au plus égal à environ 11, de préférence à 10, plus préférentiellement à 8.

Dans la présente description le terme "environ" est employé pour mettre en exergue le fait que les valeurs qui le suivent correspondent à des arrondis mathématiques et notamment qu'en l'absence de virgule, lorsque le, ou les, chiffres les plus à droite d'un nombre sont des zéros, ces zéros sont des zéros de position et non des chiffres significatifs, sauf bien entendu s'il en est précisé autrement.

En ce qui concerne l'échange, la valeur supérieure limite n'a d'importance qu'économique. Mais cette valeur basse de la limite supérieure a une influence sur l'addition de l'eau ainsi que cela sera exposé plus loin.

En effet, les études qui ont mené à la présente invention, ont montré qu'à partir du rapport de 4, l'acide fluorhydrique joue un rôle de protection vis-à-vis des réactions secondaires mettant en oeuvre le fluorure de carbamoyle. Si l'on se place en dessous de ces valeurs, on observe une quantité importante de réactions secondaires, notamment formation de biuret, réaction pour former des lourds par condensation de l'halogénure d'acyle sur le noyau aromatique, etc.

Il convient de signaler que la contrainte relative au rapport 4 est stricte et que si l'on se place en dessous ou, si au cours de la réaction le rapport descend en dessous de cette valeur, il convient de rajouter de l'acide fluorhydrique pour maintenir au cours de la réaction ce rapport supérieur à 4.

Ainsi, par exemple, si on introduit dans l'acide fluorhydrique du fluorure de carbamoyle sous la forme d'un isocyanate, et qu'il y ait 3 atomes de chlore à échanger avec le fluor, il faudra ajouter suffisamment d'acide fluorhydrique liquide pour que cette valeur soit observée tout au long de la réaction. Cette addition pourra aussi bien être réalisée dès le départ qu'au fur et à mesure de l'échange. La première option est la plus simple.

Le plus simple dans le cas qui précède, est d'observer un rapport total de l'ordre de 8, à savoir 4, pour assurer les réactions d'échange et d'addition sur la fonction isocyanate du substrat, et 4 pour assurer un bon rapport entre fluorure de carbamoyle et acide fluorhydrique.

Selon la présente invention, et compte tenu des contraintes occasionnées par une valeur inférieure ou égale à environ 20 du rapport entre le fluorure de carbamoyle et l'acide fluorhydrique (HF/substrat), il a été montré qu'il était préférable que l'addition d'eau intervienne seulement à des phases précises du procédé.
Si l'on veut maîtriser la quantité d'eau dans le milieu, l'acide fluorhydrique utilisé est bien sur essentiellement anhydre. On peut toutefois envisager l'addition d'eau sous forme d'acide fluorhydrique légèrement hydraté. Les quantités d'eau visées dans la suite de la description en vue de l'hydrolyse, comprennent les éventuelles quantités d'eau présente en tant qu'impureté dans l'acide fluorhydrique. En général, initialement avant l'étape d'hydrolyse, on utilise des acides fluorhydriques qui comportent au total au plus 0,1 QS (par rapport à l'hydrolyse de la fonction isocyanate ou fluorure de carbamoyle), avantageusement au plus 0,05, de préférence au plus 0,02 QS d'eau.

Ainsi, il est préférable d'attendre qu'il y ait au plus un atome de chlore à échanger avec l'atome de fluor pour introduire l'eau. Le moment du début d'introduction de l'eau joue un rôle non négligeable sur le rendement final (voir exemples 3 et 4 où l'on observe un écart d'environ 20 points de rendement).

On peut, en effet, suivre la réaction par rapport à l'acide chlorhydrique qui se dégage du milieu, surtout lorsqu'on utilise l'acide fluorhydrique pur liquide et lorsqu'il ne reste plus qu'au plus un seul chlore à échanger avec le fluor, on peut commencer à envisager d'ajouter l'eau qui servira à hydrolyser le fluorure de carbamoyle et à prévenir le dégagement de fluorophosgène. En effet, en l'absence d'eau il y a d'autant plus de dégagement de fluorophosgène que le milieu réactionnel est exposé longtemps à des températures supérieures à 30°C, surtout supérieures à 50°C. Rappelons que le fluorophosgène constitue un gaz très agressif et une impureté fort gênante dans les acides gazeux et surtout l'acide chlorhydrique.
Ainsi l'introduction de l'eau à un double effet :
· au niveau de l'avancement de la réaction, il convient d'attendre que l'échange soit aussi complet que possible pour maximiser le rendement,
· au niveau de la température, il est préférable que l'introduction de l'eau intervienne de manière à minimiser le temps passé en l'absence d'eau par le milieu réactionnel à des températures supérieures à 50°C.

Il est même préférable, avant de procéder à l'addition d'eau, d'attendre qu'il ne reste plus que 0,5 et même 0,2 atome de chlore à échanger par molécule. L'idéal est même d'attendre que la réaction d'échange soit complète ou quasi complète. Par complète ou quasi complète, on entend qu'il n'y a comme halogène à échanger résiduel, qu'au plus 0,02 chlore par molécule de substrat ; cela correspond au fait que dans le milieu ou dans le ciel du réacteur, et au cours du quart d'heure ultérieur, la libération de l'acide halohydrique par échange avec le fluor ne soit plus décelable par les moyens usuels de mesure. Comme exemple de moyens usuels de mesure, on peut citer la variation de pression (exempli gratia : en cas de pression autogène), ou de volume (exempli gratia en cas de pression constante), ou plus généralement variation du produit PV, ou bien enfin le dosage des substrats non complètement échangés résiduels par exemple par CPV (chromatographie phase vapeur)

Ainsi, dans la phase d'échange, selon un mode préféré de mise en oeuvre de la présente invention, on soumet le substrat, halogéné par des plus lourds que le fluor, en général chloré, à un échange, à une température au plus égale à 50°C, avantageusement au plus égale à 40°C, de préférence à une température au plus égale à 20°C, plus préférentiellement à une température inférieure au point d'ébullition de l'acide fluorhydrique liquide, et ce pendant une durée suffisante pour obtenir un échange ne laissant qu'au plus un atome de chlore à échanger par molécule.

Il est souhaitable que cette première phase soit menée à une température au moins égale à -20°C, avantageusement, à -10°C, de préférence à -5°C.

Ainsi cette première phase est avantageusement menée à une température comprise dans l'intervalle fermé [-10°C, 40°C], avantageusement dans l'intervalle fermé (c'est-à-dire comprenant les bornes) de température comprise entre 0 et 40°C ([0, 40]), de préférence la réaction se termine à une température choisie de 20°C à 40°C.

Une fois que l'échange partiel, qui ne laisse qu'au plus un atome de chlore à échanger (mais il est souhaitable qu'il n'en reste qu'au plus 0,2, avantageusement au plus 0,1, de préférence au plus 0,02), a été atteint, après avoir éventuellement porté le mélange réactionnel à une température comprise entre 30°C et 50°C, l'on commence à ajouter de l'eau, avantageusement progressivement, la chaleur dégagée par l'hydrolyse, éventuellement jointe à un chauffage extérieur, permet à ce moment là d'augmenter la température et l'on atteint une température comprise entre environ 70 et environ 100°C, en général entre 80 et 90°C.

Si l'on veut éviter au maximum l'émission de fluorophosgène, il est préconisé d'ajouter l'eau à basse température. Ainsi l'addition de l'eau commence à une température au plus égale à 50°C, avantageusement au plus égale à 30°C, de préférence 25°C.

L'addition d'eau étant terminée, on peut maintenir le mélange réactionnel dans ce dernier domaine de température jusqu'à ce que le dégagement de gaz carbonique cesse, mais il est préconisé de se placer à une température au moins égale à 70, avantageusement à 80, de préférence à 90°C et au plus égale à environ 150°C, avantageusement à 130°C, de préférence à 120°C. La durée de cette phase de finition dépend de la température de cette phase. Comme usuel, plus la température est élevée, plus courte est la durée qui permet de parfaire l'obtention du produit. A titre indicatif à 100°C une durée de 5 à 8 heures permet d'obtenir d'excellent rendement.

Selon la présente invention, dans une mise en oeuvre en discontinu, on considère que le dégagement de gaz carbonique cesse lorsque dans la composition des gaz du ciel de réacteur, la proportion de gaz carbonique ne s'élève pas de plus de 1% (en absolu) pendant % d'heure ; si l'on opère à 70°C il convient de multiplier la durée par deux.

Quoique l'on puisse utiliser des quantités plus élevées d'eau (jusqu'à 3, voire 5 équivalents, ou QS), on utilise en général au plus environ 2 équivalents. Compte tenu du fait que la présence d'eau peut gêner les opérations de recyclage et/ou de retraitement éventuel de l'acide fluorhydrique pour l'obtenir anhydre, la quantité d'eau utilisée est limitée et avantageusement comprise entre 1 et 1,5 fois la quantité stoechiométrique, de préférence entre 1,05 et 1,4 fois la quantité stoechiométrique, la valeur optimale se situe entre 1,1 et 1,2.

Lorsque l'on utilise l'isocyanate comme produit de départ, ce qui est le cas le plus fréquent, il convient de l'ajouter dans l'acide fluorhydrique : cela permet une meilleure observation de la limite basse du rapport HF/substrat.

Pour éviter toute réaction parasite, il est très important que la réaction de formation du fluorure de carbamoyle par réaction de l'acide fluorhydrique sur l'isocyanate se fasse par addition de l'isocyanate dans l'acide fluorhydrique.

Cette addition se fait avantageusement à une température inférieure à 10°C.

L'homme du métier remarquera que cette opération d'addition d'isocyanate dans l'acide fluorhydrique est parfaitement cohérente avec la contrainte de maintenir un rapport élevé entre l'acide fluorhydrique et l'isocyanate. En effet, si l'on fait le mélange dans le sens inverse, on passe par des concentrations qui ne respectent pas cette contrainte.

La réaction peut être menée sous pression et notamment sous celle qui est autogène au mélange réactionnel. En effet, l'acide fluorhydrique a un point d'ébullition suffisamment bas pour qu'aux températures de réaction, selon la pression, il puisse passer partiellement ou totalement en phase gazeuse. Aussi, dans les phases (sens temporel) où l'on opère à température supérieure à l'ébullition, est-on obligé de maintenir une pression plus élevée que la pression atmosphérique.

Une autre solution consiste à mener la réaction en présence d'un solvant. On peut notamment utiliser des solvants aromatiques, surtout des solvants aromatiques appauvris en électrons, pour éviter des réactions entre le fluorure de carbamoyle et le noyau aromatique du solvant. Conviennent bien à ce type de dilution, les halogénoaromatiques tels que les différents mono-, di- ou trichlorobenzènes.

Toutefois l'intérêt de l'utilisation de ces solvants n'est pas suffisant pour qu'ils soient utilisés systématiquement ; cette utilisation n'apporte un intérêt significatif que dans la perspective d'étape ultérieure.

Des solvants aprotiques polaires peuvent également donner de bons résultats. Mais ils ne sont pas préférés.

Si l'on résume ce qui a été développé précédemment, le procédé optimum en discontinu comporte 3 phases (sens temporel), à savoir :
· une phase d'échange à relativement basse température (voir supra),
· une phase de montée en température, phase dans laquelle est en général située l'addition d'eau (voir supra),
· une phase de finition à relativement haute température (voir supra).

Ainsi, selon une mise en oeuvre particulièrement avantageuse de la présente invention, le procédé consiste :
a) à ajouter l'isocyanate ou l'halogénure, avantageusement fluorure de carbamoyle, dans de l'acide fluorhydrique liquide, avantageusement si l'on opère à pression atmosphérique à une température au plus égale à environ 10°C (environ est placé ici pour indiquer que l'on se trouve en face d'arrondi mathématique) ;
b) éventuellement, une fois l'addition réalisée, on porte progressivement le mélange réactionnel à une température comprise entre l'ambiante (environ 20°C) et environ 40°C ;
c) le mélange réactionnel est maintenu dans le domaine de température de l'étape précédente (a ou b), pendant une durée suffisante pour qu'il n'y ait qu'au plus 1 seul, avantageusement au plus 0,1 atome de chlore à échanger avec le fluor ;
d) le mélange réactionnel est ensuite porté à une température supérieure à 40°C pendant que l'on ajoute progressivement l'eau. Cette eau provoque une augmentation de la température qui, jointe à l'opération de chauffage, conduit le mélange réactionnel à une température comprise dans l'intervalle [70, 150], plus souvent l'intervalle [70, 100]°C (2 chiffres significatifs). Le mélange réactionnel est maintenu à cette température jusqu'au moment où la réaction est finie, ce que l'on détermine par l'absence de dégagement de gaz carbonique.

Le procédé peut également être mis en oeuvre en continu ou dans un réacteur «piston».

Si l'on opère en continu, il est souhaitable d'utiliser au moins 2 réacteurs :
· dans le premier, on met en oeuvre la première phase, à savoir le mélange et l'échange ; substrat et réactifs y sont introduits, avantageusement en continu, le temps de séjour dans le réacteur est avantageusement réglé de manière qu'il ne reste plus qu'au plus 0,2, de préférence qu'au plus 0,1 atome d'halogène à échanger, avant de changer de réacteur, le domaine de température à observer est celui tel que défini pour l'introduction et l'échange ;
· dans le second réacteur, lorsqu'il n'y en a que deux, l'on introduit l'eau et l'on parfait la réaction à une température au moins égale à 50°C, avantageusement à 70°C, de préférence à 90°C, voire à 100°C, et en général au plus égale à 170°C, avantageusement à 150°C, de préférence à 130°C.

Il peut être avantageux de prévoir un troisième réacteur dans lequel l'eau est introduite et qui sera situé entre le premier et le second ; dans ce troisième réacteur, dont la température sera située entre la température du premier réacteur et du «second», avantageusement entre 40 et 130°C, aura lieu le début de l'hydrolyse, et donc l'introduction de l'eau ; hydrolyse qui sera parfaite dans ledit «second» réacteur. Cette adjonction d'un réacteur intermédiaire entre le premier et le second permet notamment de travailler dans la fourchette haute de la température de finition (avantageusement de 90 à 150°C, de préférence de 100 (2 chiffres significatifs) à 130°C).

Selon un autre mode de réalisation de l'invention, le procédé peut réaliser dans un réacteur de type piston, lequel se comporte en théorie comme une infinité de réacteur élémentaire de volume δV se déplaçant dans un réacteur en général de section constante, comme un piston dans un cylindre et subissant au fur et à mesure de son déplacement les conditions, les additions et les transformations que subit le milieu réactionnel d'un réacteur fonctionnant en discontinu.

La forme de ces réacteurs pistons est en général cylindrique, ce qui implique une section circulaire mais l'on peut imaginer d'autres sections (par exemple, elliptique ou polygonale). Ainsi, par exemple à l'entrée, le milieu réactionnel est constitué d'acide fluorhydrique, de l'éventuel solvant et du substrat, lorsqu'il a subi un déplacement correspondant au temps nécessaire à un rechange suffisant (voir supra), il entame alors au cours de son déplacement la phase de montée en température au cours de laquelle l'eau est ajoutée, la suite du déplacement du milieu correspond alors à l'étape de finition. Pour tenir compte de l'existence d'une phase gazeuse aux pressions de travail les plus courantes, il est préférable de prévoir que la partie supérieure du réacteur soit gazeuse et donc que le réacteur ne soit complètement rempli de liquide, ce qui implique que seule la partie liquide du réacteur pourra s'assimiler peu ou prou à un réacteur piston.

Ainsi que cela été mentionné au début de la présente description, la présente invention vise essentiellement comme substrat les isocyanates et les dérivés d'isocyanate (halogénure de carbamoyle) présentant à la fois un noyau arylique porteur de l'azote de la fonction isocyanate ou de la fonction halogénure de carbamoyle, et un atome de carbone, de nature sp³, perhalogéné, en général perchloré, en vue de le transformer en carbone perfluoré.

Ce carbone doit être situé en position proche d'un doublet réputé stabiliser les carbocations (sans que le mécanisme de la réaction implique nécessairement la formation d'un carbocation ; la Demanderesse connaît mal les mécanismes de cet échange). Ce doublet peut aussi bien être une insaturation et surtout l'on vise ici des carbones de nature sp³ qui sont en position benzylique par rapport à un noyau aromatique et, en particulier, les carbones sp³ directement liés au noyau arylique de la définition.

Le doublet peut également être fourni par un atome de chalcogène ou être situé en alpha d'une double liaison susceptible d'activer l'échange chlore/fluor sur ledit atome sp³. Ce type de conjugaison est explicité dans la demande de brevet européenne EP 729 930 au nom de la Demanderesse. En ce qui les concerne, les dérivés activables par la présence d'une insaturation, ou plus généralement d'une double liaison, sont décrits dans la demande WO 97/43231.

Selon la présente invention, il est hautement souhaitable que le noyau aromatique porteur de l'azote de la fonction isocyanate ou de la fonction halogénure de carbamoyle, ne porte pas de fonctions nitrées. En effet, les fonctions nitrées, ou nitro, gênent la réaction et conduisent à beaucoup de sous-produits.

Plus généralement, il est préférable que la molécule substrat ne comporte aucune fonction nitro. Le carbone aliphatique à substituer est en général un trihalogénométhyle qui conduira à un trifluorométhyle.

Le nombre de carbones total de la molécule substrat est avantageusement au plus égal à 25, de préférence à 15.

Il est préférable que le noyau aromatique soit appauvri en électrons, c'est-à-dire que compte non tenu de la fonction azote engagée dans la fonction isocyanate ou dans les fonctions en dérivant, la somme des constantes de Hammett σp des substituants dudit noyau, soit supérieure à 0, avantageusement supérieure ou égale à 0,15, plus préférentiellement à 0,25.

Pour les constantes de Hammett, on pourra se référer au livre de référence sur la chimie organique, à savoir Advanced Organic Chemistry, 3ème édition, de Jerry March, publié par John Wiley and Sons, en 1985.

Le carbone sp³ porteur des halogènes à échanger comporte au moins 2 halogènes.

Les substrats utilisables, sous leur forme de fluorure de carbamoyle, selon la présente invention, peuvent préférentiellement être de formule :

(R)ₘ-Ar(-Z-(CX₂)ₚ-GEA)-NH-CO-F

Dans cette formule, la partie aniline correspondante a pour formule :

(R)ₘ-Ar(-Z-(CX₂)ₚ-GEA)-NH-H

où :
□ Ar est un noyau aromatique, de préférence homocyclique ;
□ les X, semblables ou différents, représentent un fluor ou un radical de formule CₙF₂ₙ₊₁, avec n entier au plus égal à 5, de préférence à 2 ;
□ p représente un entier au plus égal à 2 ;
□ GEA représente un groupe hydrocarboné, un groupe électro-attracteur dont les éventuelles fonctions sont inertes dans les conditions de la réaction, avantageusement fluor, ou un reste perfluoré de formule CₙF₂ₙ₊₁, avec un entier au plus égal à 8, avantageusement à 5 ;
□ le nombre total de carbone de -(CX₂)ₚ-GEA est avantageusement compris entre 1 et 15, de préférence entre 1 et 10 ;
□ m est 0 ou un entier choisi dans l'intervalle fermé (c'est-à-dire comprenant les bornes) 1 à 4 ;
□ R est un substituant inerte dans les conditions opératoires, avantageusement choisi parmi les halogènes avantageusement légers (c'est à dire chlore et fluor) et les radicaux hydrocarbonés, de préférence alcoyle, aryle, alcoylchalcogényle (tel que alcoyloxyle), arylchalcogényle (tel que aryloxyle) ;
□ Z représente une liaison simple, un atome de chalcogène avantageusement léger (soufre et oxygène).

Il est préféré que Ar soit avantageusement monocyclique, de préférence à 6 chaînons.

En particulier l'aniline correspondant au substrat peut répondre à la formule : 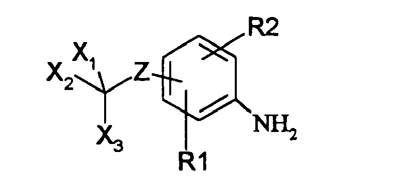 où :
□ Z représente une simple liaison, ou un atome de chalcogène, où X₁, X₂, X₃ représentent des halogènes semblables et différents, avec la condition qu'au moins 2 halogènes soient différents du fluor.
□ R₁ et R₂ sont des substituants parmi les halogènes, les alcoyles, les aryles, les nitriles.

Le radical X₃ peut être un groupe électro-attracteur n'interférant pas avec la réaction, et notamment peut être un groupe perfluoré, généralement désigné dans le domaine de la technique R_{f}.

Le composé substrat peut notamment être de la formule (les substituants éventuels inertes dans les conditions réactionnelles ne sont pas figurés) des composés des équations ci après :

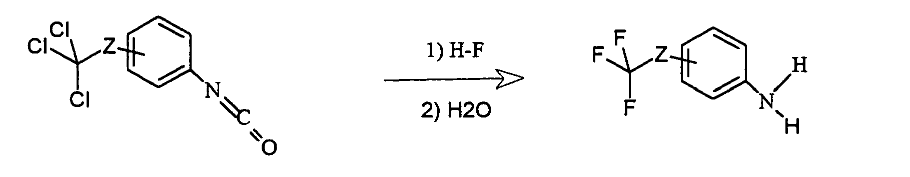

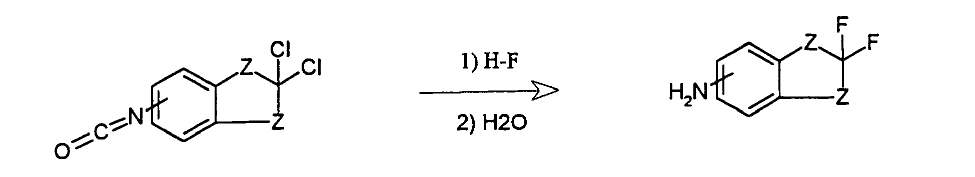

Dans la 2^{ème} équation, les Z, semblables ou différents, représentent un chalcogène avantageusement légers (soufre et surtout oxygène).
Les exemples non limitatifs suivants illustrent l'invention.

### Exemple 1

On introduit dans un réacteur de l'HF (9 moles) à une température de -5°C. On introduit alors le trichlorométhylphénylisocyanate (1 mole) que l'on désire transformer, la fluoration dure 1 heure 30, à une température de 20°C.

Une opération de finition est menée à la température de 45°C pendant 2 heures. On observe qu'il reste alors moins de 0,1 atome de chlore à échanger.

On ajoute l'eau (1,05 mole) ; en 1 heure la température s'élève à 80°C.

On chauffe alors pour porter et maintenir la température à 90°C pendant 2 heures.

Le rendement isolé après neutralisation et distillation est de 93%.

### Exemple 2 - (comparatif) effet de l'insuffisance de HF

On introduit dans un réacteur de l'HF (3,45 moles) à une température de -0°C. L'HF est chauffé à 10°C ; on introduit alors le trichlorométhylphénylisocyanate (0,54 mole, 127,7 g) que l'on désire transformer, la fluoration dure 2 heures à une température de 10°C.

Ainsi, lors de l'introduction, le rapport HF/fluorure de carbamoyle est égal à 5,4, après l'échange complet, il n'est plus que de 2,4.

Le milieu réactionnel est chauffé à 100°C, pendant la montée en température entre 70 et 80°C, 0,81 mole d'eau est introduit. La réaction d'hydrolyse est poursuivie 1 heure et 30 min. à 100°C. Puis le milieu réactionnel est refroidi à 10°C, coulé sur un mélange eau et glace, neutralisé par une solution aqueuse de potasse, la pTFMA est extraite au dichlorométhane. La solution de dichlorométhane contient 52 g de pTFMA, soit un rendement de 60%.

### Exemple 3 - rôle du moment où l'on introduit l'eau

On introduit dans un réacteur de l'eau (0,614 mole) et de l'HF (5,05 moles) à une température de 0°C. Le mélange est chauffé à 10°C. On introduit alors, en environ 15 minutes, le trichlorométhylphénylisocyanate (0,414 mole, 97.9 g) que l'on désire transformer en maintenant la température à 10°C. La fluoration est poursuivie 1 heure à une température de 10°C.

Le milieu réactionnel est chauffé à 100°C. La réaction d'hydrolyse est poursuivie 1 heure à 100°C. Puis le milieu réactionnel est refroidi à 10°C, coulé sur un mélange eau et glace, neutralisé par une solution aqueuse de potasse, la pTFMA est extraite au dichlorométhane. La solution de dichlorométhane est analysée par HPLC, elle contient 49 g de pTFMA, soit un rendement de 73,5%.

### Exemple 4

On introduit dans un réacteur de l'HF (6,62 moles) à une température de -0°C. L'HF est chauffé à 10°C. On introduit alors, en environ 15 min., le trichlorométhylphénylisocyanate (0,544 mole, 128.6 g) que l'on désire transformer, la fluoration dure 1 heure à une température de 10°C.

Le milieu réactionnel est porté progressivement à 100°C. Lorsque la température à atteint 70°C, on vérifie que l'échange est complet (c'est-à-dire que le CI résiduel est inférieur à 2% en équivalent). On introduit 0,88 mole d'eau pendant la montée en température entre 70 et 90°C. La réaction d'hydrolyse est poursuivie 1 heure à 100°C. Puis le milieu réactionnel est refroidi à 10°C, coulé sur un mélange eau et glace, neutralisé par une solution aqueuse de potasse, la pTFMA est extraite au dichlorométhane. La solution de dichlorométhane est concentrée par évaporation du dichlorométhane. On obtient 92.3 g d'une huile contenant 93,7% de pTFMA, soit un rendement supérieure à 98,6%.

## Revendications

1. Procédé de synthèse de composés comportant à la fois un carbone perfiuoré et une fonction aniline dans lequel on traite un fluorure de carbamoyle arylique, comportant dans sa molécule au moins un carbone aliphatique perhalogéné en position proche d'un doublet réputé stabiliser les carbocations et lequel porte au moins 1, avantageusement au moins 2 atomes d'halogène à échanger avec le fluor, par mise en contact dudit fluorure de carbamoyle arylique avec de l'acide fluofiydrique et de l'eau en phase liquide, **caractérisé par le fait qu'**il comporte l'étape de mise en contact dudit fluorure de carbamoyle avec de l'acide fluorhydrique, le rapport acide fluorhydrique sur fluorure de carbamoyle étant maintenu pendant la réaction à une valeur au moins égale à 4, de préférence à 5, et que ledit rapport est au plus égal à 11 et **par le fait que** l'addition de l'eau au mélange réactionnel n'intervient qu'après qu'il ne reste qu'au plus un seul halogène par molécule de substrat à échanger avec le fluor.

2. Procédé selon la revendication 1 **caractérisé par** la fait que le rapport acide fluorhydrique sur fluorure de carbamoyle est au moins égal à 6.

3. Procédé selon l'une des revendications 1 et 2 **caractérisé par le fait que** le rapport acide fluorhydrique sur fluorure de carbamoyle est au plus égal à 10, plus préférentiellement à 8.

4. Procédé selon l'une des revendications 1 à 3, **caractérisé par le fait que** ledit fluorure de carbamoyle est formé in situ par addition d'un isocyanate dans l'acide fluorhydrique liquide, à une température au plus égale à 10°C.

5. Procédé selon les revendications 1 à 4, **caractérisé par le fait que** la température est maintenue à une valeur au plus égale à 40°C jusqu'au moment où il ne reste qu'au plus un atome d'halogène à échanger avec la fluor, avantageusement au plus 0,1 atome.

6. Procédé selon les revendications 1 à 5, **caractérisé par le fait que** l'addition de l'eau intervient à une température comprise entre 10°C et 90°C.

7. Procédé selon la revendication 4, **caractérisé par le fait que** l'addition de l'eau commence à une température au plus égale à 50°C, avantageusement au plus égale à 30°C.

8. Procédé selon les revendications 1 à 5, **caractérisé par** la fait que une fois l'addition d'eau terminée, le mélange réactionnel est maintenu à une température au moins égale à 80°C et au plus égale à 130°C et ce jusqu'à ce que le dégagement de gaz carbonique cesse.

9. Procédé selon les revendications 1 à 6, **caractérisé par le fait que** la quantité d'eau introduite dans le mélange réactionnel est égale à une valeur comprise entre 1 fois et 1,5 fois la quantité stoechiométrique nécessaire pour l'hydrolyse de la fonction fluorure de carbamoyle.

10. Procédé selon l'une des revendications 1 à 5, 8 et 9 **caractérisé par le fait que** le procédé est réalisé en continu et que, quand on introduit l'eau, la température du milieu réactionnel est au moins égale à 70°C, avantageusement à 90°C, de préférence à 100°C.

11. Procédé selon les revendications 1 à 5, 8 et 9, **caractérisé par le fait que** le procédé est réalisé en continu et que l'on d'utilise au moins 2 réacteurs :
· dans le premier, on met en oeuvre la première phase, à savoir le mélange et l'échange ; substrat et réactifs y sont introduits, avantageusement en continu, le temps de séjour dans le réacteur est avantageusement réglé de manière qu'il ne reste plus qu'au plus 0,2, de préférence qu'au plus 0,1 atome d'halogène à échanger; avant de changer de réacteur, le domaine de température à observer est celui tel que défini pour l'introduction et l'échange ;
· dans le second réacteur, lorsqu'il n'y en a que deux, l'on introduit l'eau et l'on parfait la réaction à une température au moins égale à 50°C, avantageusement à 70°C, de préférence à 90°C, voire à 100°C et, en général, au plus égale à 170°C, avantageusement à 150°C, de préférence à 130°C.

12. Procédé selon les revendications 1 à 11, **caractérisé par le fait que** la réaction est menée dans un réacteur piston.

13. Procédé selon l'une des revendications 1 à 12 **caractérisé par le fait que** la partie aniline correspondante au fluorure de carbamoyle répond à la formule :
(R)ₘ-Ar(-Z-(CX₂)ₚ-GEA)-NH-H
où :
□ Ar est un noyau aromatique, de préférence homocyclique ;
□ les X, semblables ou différents, représentent un fluor ou un radical de formule CₙF₂ₙ₊₁, avec n entier au plus égal à 5, de préférence à 2 ;
□ p représente un entier au plus égal à 2 ;
□ GEA représente un groupe hydrocarboné, un groupe électro-attracteur dont les éventuelles fonctions sont inertes dans les conditions de la réaction, avantageusement fluor, ou un reste perfluoré de formule CₙF₂ₙ₊₁, avec un entier au plus égal à 8, avantageusement à 5 ;
□ le nombre total de carbone de -(CX₂)ₚ-GEA est avantageusement compris entre 1 et 15, de préférence entre 1 et 10 ;
□ m est 0 ou un entier choisi dans l'intervalle fermé (c'est-à-dire comprenant les bornes) 1 à 4 ;
□ R est un substituant inerte dans les conditions opératoires, avantageusement choisi parmi les halogènes avantageusement légers (c'est à dire chlore et fluor) et les radicaux hydrocarbonés, de préférence alcoyle, aryle, alcoylchalcogényle (tel que alcoyloxyle), arylchalrogényle (tel que aryloxyle) ;
□ Z représente une liaison simple, un atome de chalcogène avantageusement léger (soufre et oxygène).

14. Procédé selon l'une des revendications 1 à 12 **caractérisé par le fait que** la partie aniline correspondante au fluorure de carbamoyle répond à la formule : 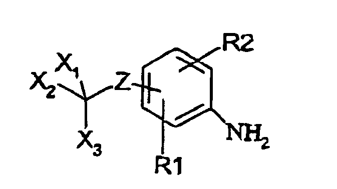 où:
□ Z représente une simple liaison, ou un atome de chalcogène, où X₁, X₂, X₃ représentent des halogènes semblables et différents, avec la condition qu'au moins 2 halogènes soient différents du fluor.
□ R₁ et R₂ sont des substituants parmi les halogènes, les alcoyles, les aryles, les nitriles.

15. Procédé selon l'une des revendications 1 à 12 **caractérisé par le fait que** la partie aniline correspondante au fluorure de carbamoyle est la p-trifluorométhylaniline.

## Claims

1. Process for synthesizing compounds comprising both a perfluorinated carbon and an aniline function, in which an arylcarbamoyl fluoride comprising in its molecule at least one perhalogenated aliphatic carbon in a position close to a lone pair known to stabilize carbocations, and which bears at least one and advantageously at least two halogen atoms to be exchanged with fluorine, is treated by placing the said arylcarbamoyl fluoride in contact with hydrofluoric acid and water in the liquid phase, **characterized in that** it includes the step of placing the said carbamoyl fluoride in contact with hydrofluoric acid, the ratio of hydrofluoric acid to carbamoyl fluoride being maintained during the reaction at a value of at least 4 and preferably 5, and **in that** the said ratio is not more than 11, and **in that** the addition of water to the reaction mixture takes place only after there is not more than one halogen per substrate molecule remaining to be exchanged with fluorine.

2. Process according to Claim 1, **characterized in that** the ratio of hydrofluoric acid to carbamoyl fluoride is at least 6.

3. Process according to either of Claims 1 and 2, **characterized in that** the ratio of hydrofluoric acid to carbamoyl fluoride is not more than 10 and more preferentially not more than 8.

4. Process according to one of Claims 1 to 3, **characterized in that** the said carbamoyl fluoride is formed *in situ* by addition of an isocyanate to liquid hydrofluoric acid, at a temperature of not more than 10°C.

5. Process according to Claims 1 to 4, **characterized in that** the temperature is maintained at a value of not more than 40°C until there is not more than one and advantageously not more than 0.1 halogen atom remaining to be exchanged with fluorine.

6. Process according to Claims 1 to 5, **characterized in that** the addition of water takes place at a temperature of between 10°C and 90°C.

7. Process according to Claim 4, **characterized in that** the addition of water begins at a temperature of not more than 50°C and advantageously not more than 30°C.

8. Process according to Claims 1 to 5, **characterized in that** once the addition of water is complete, the reaction mixture is maintained at a temperature of at least 80°C and of not more than 130°C until the evolution of carbon dioxide has ceased.

9. Process according to Claims 1 to 6, **characterized in that** the amount of water introduced into the reaction mixture is equal to a value of between 1 and 1.5 times the stoichiometric amount required for the hydrolysis of the carbamoyl fluoride function.

10. Process according to one of Claims 1 to 5, 8 and 9, **characterized in that** the process is performed continuously and **in that**, when the water is introduced, the temperature of the reaction medium is at least 70°C, advantageously 90°C and preferably 100°C.

11. Process according to Claims 1 to 5, 8 and 9, **characterized in that** the process is performed continuously and **in that** at least two reactors are used:
• in the first, the first phase is performed, i.e. the mixing and the exchange; the substrate and the reagents are introduced therein, advantageously continuously, the residence time in the reactor is advantageously controlled so that there is not more than 0.2 and preferably not more than 0.1 halogen atom remaining to be exchanged; before changing reactor, the temperature range to be observed is that as defined for the introduction and the exchange;
• in the second reactor, when there are only two, the water is introduced and the reaction is completed at a temperature of at least 50°C, advantageously 70°C, preferably 90°C or even 100°C, and generally of not more than 170°C, advantageously 150°C and preferably 130°C.

12. Process according to Claims 1 to 11, **characterized in that** the reaction is performed in a piston reactor.

13. Process according to one of Claims 1 to 12,
**characterized in that** the aniline portion corresponding to the carbamoyl fluoride corresponds to the formula:
(R)ₘ-Ar(-Z- (CX₂)ₚ-EWG) -NH-H
in which:
■ Ar is an aromatic nucleus, which is preferably homocyclic;
■ the groups X, which may be identical or different, represent a fluorine or a radical of formula CₙF₂ₙ₊₁, with n being an integer not greater than 5 and preferably not greater than 2;
■ p represents an integer not greater than 2;
■ EWG represents a hydrocarbon-based group, an electron-withdrawing group whose possible functions are inert under the reaction conditions, advantageously fluorine, or a perfluorinated residue of formula CₙF₂ₙ₊₁, with n being an integer not greater than 8 and advantageously not greater than 5;
■ the total carbon number of -(CX₂)ₚ-EWG is advantageously between 1 and 15 and preferably between 1 and 10;
■ m is 0 or an integer chosen in the closed range (i.e. including the limits) 1 to 4;
■ R is a substituent that is inert under the operating conditions, advantageously chosen from halogens that are advantageously light (i.e. chlorine or fluorine) and hydrocarbon-based radicals, preferably alkyl, aryl, alkylchalcogenyl (such as alkyloxy) and arylchalcogenyl (such as aryloxy);
■ Z represents a single bond or a chalcogen atom that is advantageously light (sulfur or oxygen).

14. Process according to one of Claims 1 to 12, **characterized in that** the aniline portion corresponding to the carbamoyl fluoride corresponds to the formula: 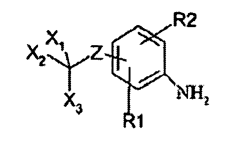 in which:
■ Z represents a single bond or a chalcogen atom, in which X₁, X₂ and X₃ represent identical or different halogens, with the condition that at least two halogens are other than fluorine;
■ R₁ and R₂ are substituents chosen from halogens, alkyls, aryls and nitriles.

15. Process according to one of Claims 1 to 12, **characterized in that** the aniline portion corresponding to the carbamoyl fluoride is p-trifluoromethylaniline.

## Patentansprüche

1. Verfahren zur Synthese von Verbindungen, welche gleichzeitlich einen perfluorierten Kohlenstoff und eine Anilinfunktion aufweisen, bei dem man ein arylisches Carbamoylfluorid, das in seinen Molekül mindestens einen perhalogenierten aliphatischen Kohlenstoff in Nachbarposition zu einer Doppelbindung, die bekanntermaßen Carbokationen stabilisiert, aufweist und das mindestens 1, vorzugsweise mindestens 2, gegen Fluor auszutauschende Halogenatome trägt, mittels Inkontaktbringen des arylischen Carbamoylfluorids mit Fluorwasserstoff und Wasser in flüssiger Phase behandelt, **dadurch gekennzeichnet, daß** das Verfahren den Verfahrensschritt des Inkontaktbringens des Carbamoylfluorids mit dem Fluorwasserstoff umfaßt, wobei das Fluorwasserstoff/Carbamoylfluorid-Verhältnis während der Reaktion bei einem Wert von mindestens 4, vorzugsweise mindestens 5, konstantgehalten wird und dieses Verhältnis höchstens 11 beträgt, und daß die Zugabe des Wassers zu der Reaktionsmischung erst erfolgt, nachdem nur noch höchstens ein einziges gegen Fluor auszutauschendes Halogen pro Ausgangsmolekül vorhanden ist.

2. Verfahren nach Anspruch 1, **dadurch gekennzeichnet, daß** das Fluorwasserstoff/Carbamoylfluorid-Verhältnis mindestens 6 beträgt.

3. Verfahren nach einem der Ansprüche 1 und 2, **dadurch gekennzeichnet, daß** das Fluorwasserstoff/Carbamoylfluorid-Verhältnis höchstens 10, vorzugsweise höchstens 8, beträgt.

4. Verfahren nach einem der Ansprüche 1 bis 3, **dadurch gekennzeichnet, daß** das Carbamoylfluorid in situ durch Zugabe eines Isocyanats in flüssigen Fluorwasserstoff bei einer Temperatur von höchstens 10 °C gebildet wird.

5. Verfahren nach einem der Ansprüche 1 bis 4, **dadurch gekennzeichnet, daß** das die Temperatur bei einem Wert von höchstens 40 °C konstantgehalten wird bis zu dem Moment, wo nur noch höchstens ein gegen Fluor auszutauschendes Halogen, vorzugsweise nur noch höchstens 0,1 Atome, vorhanden sind.

6. Verfahren nach einem der Ansprüche 1 bis 5, **dadurch gekennzeichnet, daß** die Wasserzugabe bei einer Temperatur zwischen 10 °C und 90 °C erfolgt.

7. Verfahren nach Anspruch 4, **dadurch gekennzeichnet, daß** die Wasserzugabe bei einer Temperatur von höchstens 50°C, vorzugsweise höchstens 30 °C, beginnt.

8. Verfahren nach einem der Ansprüche 1 bis 5, **dadurch gekennzeichnet, daß** nach Beendigung der Wasserzugabe die Reaktionsmischung bei einer Temperatur von mindestens 80 °C und höchstens 130 °C konstantgehalten wird, und dies, bis die Freisetzung von Kohlendioxid aufhört.

9. Verfahren nach einem der Ansprüche 1 bis 6, **dadurch gekennzeichnet, daß** die in die Reaktionsmischung eingetragene Wassermenge einem Wert zwischen dem 1fachen bis 1,5fachen der für die Hydrolyse der Carbamoylfluoridfunktion erforderlichen stöchiometischen Menge entspricht.

10. Verfahren nach einem der Ansprüche 1 bis 5, 8 und 9, **dadurch gekennzeichnet, daß** das Verfahren kontinuierlich durchgeführt wird und daß, wenn man das Wasser hinzugibt, die Temperatur des Reaktionsmilieus mindestens 70 °C, vorzugsweise mindestens 90 °C, vorzugsweise mindestens 100 °C, beträgt.

11. Verfahren nach einem der Ansprüche 1 bis 5, 8 und 9, **dadurch gekennzeichnet, daß** das Verfahren kontinuierlich durchgeführt wird und daß man mindestens 2 Reaktoren verwendet:
- In dem ersten Reaktor führt man die erste Phase, nämlich die Mischung und den Austausch, durch: Die Ausgangsverbindung und die Reagenzien werden dort eingetragen, vorzugsweise kontinuierlich, und die Verweildauer in dem Reaktor wird vorzugsweise derart geregelt, daß nur noch höchstens 0,2, vorzugsweise nur noch höchstens 0,1, auszutauschende Halogenatome verbleiben; vor dem Reaktorwechsel ist der zu beobachtende Temperaturbereich derart, wie für die Zugabe und den Austausch definiert;
- In dem zweiten Reaktor, wenn man nur zwei Reaktoren verwendet, trägt man das Wasser ein und vollendet die Reaktion bei einer Temperatur von mindestens 50 °C, vorzugsweise mindestens 70 °C, bevorzugt mindestens 90 °C, sogar mindestens 100 °C, und im allgemeinen von höchstens 170 °C, vorzugsweise höchstens 150 °C, bevorzugt höchstens 130 °C.

12. Verfahren nach einem der Ansprüche 1 bis 11, **dadurch gekennzeichnet, daß** die Reaktion in einem Kolbenreaktor durchgeführt wird.

13. Verfahren nach einem der Ansprüche 1 bis 12, **dadurch gekennzeichnet, daß** der dem Carbamoylfluorid entsprechende Anilinteil der Formel
(R)ₘ-Ar(-Z-(CX₂)ₚ-GEA)-NH-H
entspricht, wobei:
- Ar ein vorzugsweise homocyklischer aromatischer Kern ist;
- X, gleich oder verschieden, ein Fluoratom oder einen Rest der Formel CₙF₂ₙ₊₁ darstellt, wobei n eine ganze Zahl von höchstens 5, vorzugsweise höchstens 2, ist;
- p eine ganze Zahl von höchstens 2 darstellt;
- GEA eine Kohlenwasserstoffgruppe, eine elektronenziehende Gruppe, deren gegebenenfalls vorhandene Funktionen unter den Reaktionbedingungen inert sind, vorzugsweise Fluor, oder einen perfluorierten Rest der Formel CₙF₂ₙ₊₁, wobei n eine ganze Zahl von höchstens 8, vorzugsweise höchstens 5, ist, darstellt;
- die Gesamtkohlenstoffanzahl von -(CX₂)ₚ-GEA vorzugsweise zwischen 1 und 15, bevorzugt zwischen 1 und 10, liegt;
- m 0 oder eine ganze Zahl, ausgewählt im geschlossenen Intervall (d. h. einschließlich der Grenzen) von 1 bis 4 ist;
- R ein unter Verfahrensbedingungen inerter Substituent ist, vorzugsweise ausgewählt aus vorzugsweise leichten Halogenen (d.h. Chlor und Fluor) und Kohlenwasserstoffresten, vorzugsweise Alkyl, Aryl, Alkylchalkogenyl (wie Alkyloxyl), Arylchalkogenyl (wie Aryloxyl);
- Z eine einfache Bindung oder ein vorzugsweise leichtes Chalkogenatom (Schwefel oder Sauerstoff) darstellt.

14. Verfahren nach einem der Ansprüche 1 bis 12, **dadurch gekennzeichnet, daß** der dem Carbamoylfluorid entsprechende Anilinteil der Formel 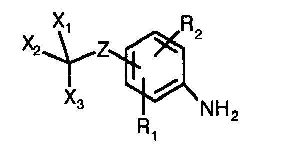 entspricht, wobei:
- Zeine Einfachbindung oder ein Chalkogenatom darstellt, wobei X₁, X₂ und X₃ gleiche oder voneinander verschiedene Halogene darstellen mit der Maßgabe, daß mindestens 2 Halogene von Fluor verschieden sind;
- R₁ und R₂ Substituenten aus Halogenen, Alkylen, Arylen und Nitrilen sind.

15. Verfahren nach einem der Ansprüche 1 bis 12, **dadurch gekennzeichnet, daß** der dem Carbamoylfluorid entsprechende Anilinteil p-Trifluormethylanilin ist.
